# EUROPEAN PATENT SPECIFICATION

(11) **EP 2 380 507 B1**
(45) Date of publication and mention of the grant of the patent: **31.10.2012**
(21) Application number: 10004383.5
(22) Date of filing: 26.04.2010
(51) Int. Cl.: A61B 17/11

(54) **Sleeve-type fixing method and device for anastomosis for tubular organs such as intestines, stomach, esophagus**
Hülsentypfixierungsverfahren und -vorrichtung zur Anastomose für Tubulusorgane, wie Eingeweide, Magen, Speiseröhre
Procédé de fixation de type manchon et dispositif d'anastomose d'organes tubulaires tels que les intestins, l'estomac et l'ésophage

(43) Date of publication of application: 26.10.2011
(73) Proprietor: Liu, Zhongchen, Xiamen Fujian (CN)
(72) Inventor: Liu, Zhongchen, Xiamen Fujian (CN)
(74) Representative: Garavelli, Paolo

(56) References cited:
- EP-A2- 0 362 163
- EP-A2- 1 797 831
- US-A1- 2004 015 179
- US-A1- 2006 085 035

## Description

The present invention relates to an anastomosis method and device for tubular organs such as intestines or stomach etc., particularly to a sleeve type fixing method and device for anastomosis such as for intestines, stomach, esophagus and gallbladder etc..

Operations of intestines and gallblade etc. are common surgery operations: especially after the total stomach removal, the intestines and esophagus must be subjected to anastommosis, but after anastomosis by existing anastomosis devices, it complications often happen, such as leakage, anastomotic stenosis, reflux, hard food passing, and the existing anastomosis device has a high cost.

Tubular type surgical staplers used in the art (such as CDH25 anastomat, Johnson & Johnson) are used to evert the distal and proximal intestines and secured them by two row metal pins; after healing, a constriction ring without flexable function is formed, thus complications again occur, such as leakage, anastomotic stenosis, reflux, hard food passing etc. after healing.

An elastic ring adapter for used in esophagus gastric-chamber, disclosed in a Chinese invention with publication number CN1036898, is particularly suitable for use in esophagogastroanastomotic operations after removal of esophageal and cardiac cancers. The applicant of the present invention has two applications: CN101327142 and CN101243987, which provide devices for intestines anastomosis, without intestinal anastomotic leakage, anastomotic stenosis and foreign body residue at the anastomotic and with few reflux, smooth passing of the holding substance and low medical service cost, but the operation is complex and needs long time for surgery, and the anastomosis is not well enough

US A1-2006/085035, EP-A2-1 797 831, US-A1-2004/015179 and EP-A2-0362163 disclose devices for anastomosis according to the prior art.

Object of the present invention is providing a method and device for anastomosis such as for intestines, stomach, esophagus and gallbladder, which has a simple structure, a simple operation and a good fixation.

A sleeve type fixing method and device for anastomosis for tubular organs such as for intestines, stomach, esophagus etc., of the invention are disclosed in the releted independent claims.

In particular, the device comprises an inner ring and an outer ring, wherein said outer ring is made of elastic material, two intestines ends are respectively sewed to said outer ring and inner ring, and fixed to the outside of the rings, both the outer ring and the intestine fixed on said outer ring are overturned so that the intestine is connected to the inside of the outer ring, and the outer ring and inner ring and the adventitia of the intestines fixed on the rings are clamped together.

Further, the upper portion of said ring is used for anastomose, the middle portion is used for connecting, and the lower portion is used for insertion, the diameter of the connecting portion is smaller than the insertion portion and anastomose portion, the outer ring is made of elastic material, the inside of the lower portion of the outer ring has an inwardly protruding ring, and said protruding ring is clamped to the connecting portion of the inner ring.

Said insertion portion of said inner ring is provided with axial grooves so as to narrow the diameter of the insertion portion.

The bottom of the insertion portion is provided with barbs to prevent the outer ring from being separated.

Said insertion portion of the inner ring is provided with a flange around the upper portion.

The sleeve type method for anastomosis for the intestines of the present invention is achieved by an anastomosis device which comprises an inner ring and an outer ring,the upper portion is anastomosis portion, the middle portion is connecting portion and the lower portion is insertion portion, wherein the diameter of the connecting portion is smaller than the insertion portion and the anastomosis portion, and said outer ring is made of elastic material; the inside of the lower portion of the outer ring has an inwardly protruding ring, and said protruding ring is clamped to the connecting portion of the inner ring; in operation, firstly one intestine is sleeved on the flange of the anastomosis portion of said inner ring and fixed, and the outer ring is overturned so as to let the lower edge to overturn, then another intestine is sleeved on the outer ring and is fixed, then both the outer ring and the intestine are overturned so that the intestine is connected in the inner edge of the outer ring, finally said inner ring is inserted, and the two intestines are overlapped on the anastomosis portion of the inner ring and outer ring.

The present invention is to fix the intestines to the outer ring, let the intestine and the outer ring to overturn inwardly to let the intestine to fixed in the inner side of the outer ring by the elastic material and structure of the outer ring, so as to connect to another intestine fixed on the inner ring: this operation is simple and has a good effect. And the invention is fit for anastomosis between intestines, intestine and stomach, esophagus and intestine, esophagus and stomach, bile duct and intestine etc..

The present invention will be better described by some preferred embodiments thereof, provided as a non-limiting example, with reference to the enclosed drawings, in which:
FIG. 1 is an exploded perspective view of the present invention;
FIG. 2 is a sectional view of the inner ring;
FIG. 3 is a sectional view of the outer ring;
FIG. 4 shows an intestine sleeved on the inner ring;
FIG. 5 is a sectional view of FIG. 4;
FIG. 6 shows the outer ring sleeved an intestine after being overturned inwardly;
FIG. 7 shows the outer ring overturned outwardly after being sleeved on an intestine;
FIG. 8-1 is step 1 of a flow for using the outer ring;
FIG. 8-2 is step 2 of the flow for using the outer ring;
FIG. 8-3 is step 3 of the flow for using the outer ring;
FIG. 8-4 is step 4 of the flow for using the outer ring;
FIG. 8-5 is step 5 of the flow for using the outer ring;
FIG. 9 shows the intestines is anastomosis and fixed;
FIG. 10 is a perspective view of the outer ring in another embodiment;
FIG. 11 is a sectional view of the outer ring in another embodiment.

The above-mentioned and other features and advantages of this invention, and the manner of attaining them, will become more apparent and the invention itself will be better understood with reference to the following description of embodiments of the invention taken in conjunction with the accompanying drawings.

### Embodiment 1

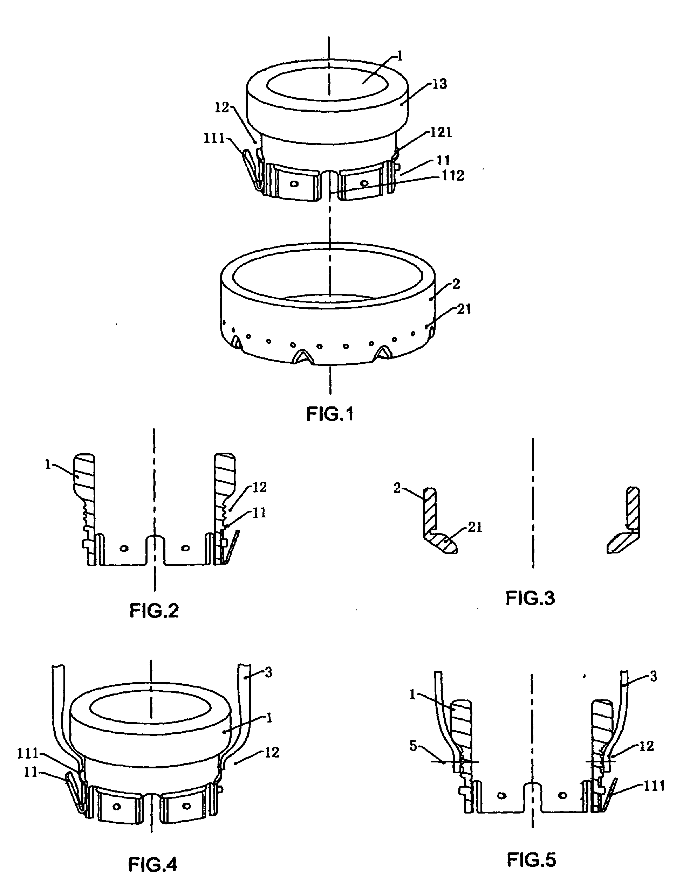

Referring to FIG. 1 to 3, the anastomosis device comprises an inner ring 1 and an outer ring 2; the upper portion of the inner ring is an anastomosis portion 13, the middle portion is a connection portion 12 and the lower portion is an insertion portion 11.

The diameter of the connecting portion 12 is smaller than the insertion portion 11 and the anastomosis portion 13, the outer ring 2 is made of elastic material, and the side wall of the outer ring 2 is provided with thread holes 21 set according to the sewing position of the intestines; the bottom of the outer ring is made as a protruded ring 21, the protruded ring 21 is clamped to the connecting portion 12 of the inner ring, and the lower edge of the connecting portion of the inner ring is formed as a circle or several flanges 121. The insertion portion 11 of said inner ring has axial grooves 112, thus the diameter of the insertion portion is variable and narrowing. The insertion portion 11 has barbs 111 which can prevent the outer ring from being separated.

The inner ring 1 can be made of food-grade hard plastic, and the height of the inner ring 1 is between 3 and 6mm.

The outer ring 2 can be made of nitrile-butadiene rubber, and the height of the outer ring is between 3 and 6mm.

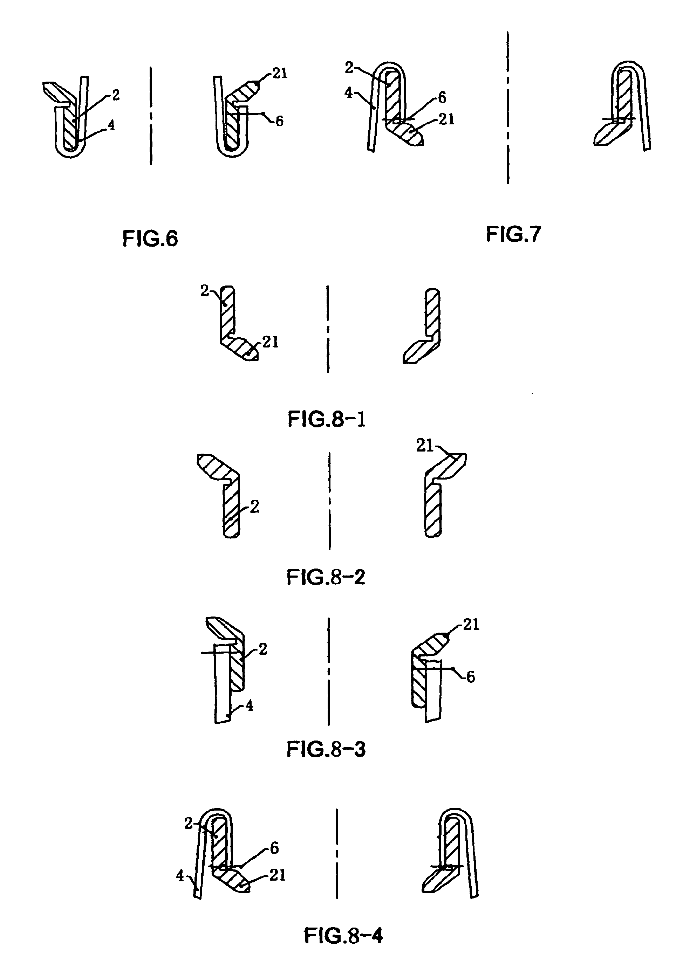

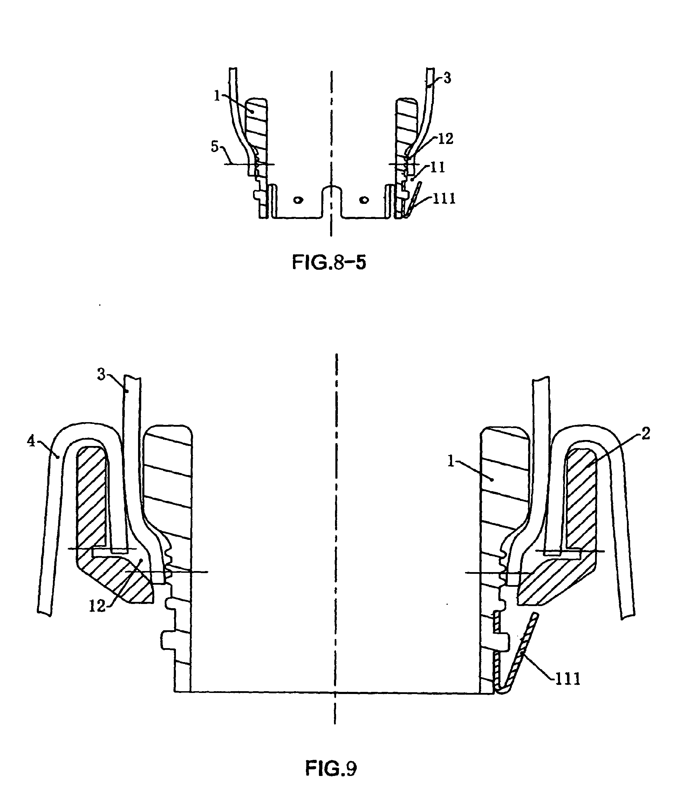

Referring to FIG. 4 to 8, in operation, firstly, an intestine 3 is sleeved on the outside of the anastomosis portion of the inner ring 1; the intestine can be sewed in the grooves of the connecting portion 12 of the inner ring by an absorbable thread 5; as shown in FIG. 8-1 and, 8-2, the outer ring 2 is overturned inwardly and the inward flange 21 is placed as to be outward, then another intestine 4 is sleeved on the outer ring 2 and is fixed by an absorbable thread. Then, both the outer ring 2 and the fixed intestine 4 are overturned to let the connecting end of the intestine 6 in the inner side of the outer ring 2; finally, the inner ring 1 is inserted, then two intestines are overlapped in the anastomosis portion: the inner ring 1, outer ring 2 and two intestines 3,4 are formed as a stable fixation structure, the serous membranes of the intestines are contacted with each other, and the width of the contacting area is about 0.4 - 0.5 cm.

Usually, the inner ring 1 and outer ring 2 will be automatically detached from the intestines in about 10 days, and discharged out from the anus, the intestine and the esophagus is healed in sleeve type, the healing is without intestinal anastomotic leakage, anastomotic stenosis and foreign body residue at the anastomotic and with few reflux, smooth passing of the holding substance and low medical service cost.

### Embodiment 2 (not part of the invention)

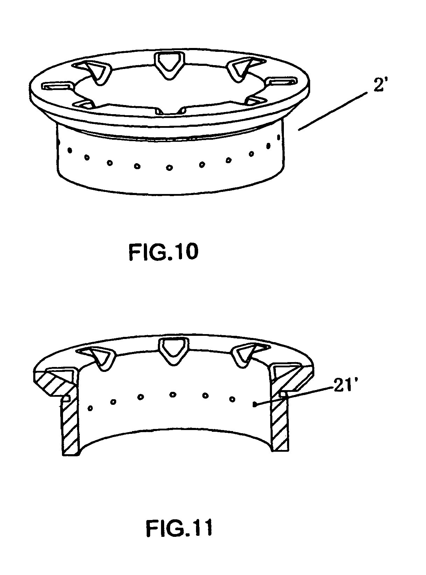

In this embodiment, the outer ring is different from embodiment 1; as shown in FIG. 10 and FIG. 11, the outer ring Z' is extended out a ring Z1' in the inner side of the outer ring Z', the inner ring 1 has the same structure as the embodiment 1, so in operation, the inner ring do not need to be overturned, the intestines are sewed in the outer ring Z' and then overturned, and the other process is the same as embodiment 1.

## Claims

1. Sleeve-type fixing device for anastomosis for tubular organs, such as for intestines, stomach, esophagus, comprising an inner ring (1) and an outer ring (2), wherein said outer ring (2) is made of elastic material, said outer ring (2) and said inner ring (1) are adapted to be sewed to two intestines ends (3, 4) and to be fixed to the two intestines ends (3, 4) on an external part of the outer ring (2) and the inner ring (1), **characterised in that** the outer ring (2) when the intestine (4) is fixed on said outer ring (2) is adapted to be overturned so that the intestine (4) is connected to the inside of the outer ring (2), the outer ring (2) and the inner ring (1) and the adventitia (3, 4) of the intestines when fixed on the rings (1, 2) being adapted to be clamped together.

2. Sleeve-type fixing device according to claim 1, wherein the upper portion (13) of the inner ring (1) is used for anastomose, the middle portion (12) is used for connecting, and the lower portion (11) is used for insertion, the diameter of the connecting portion (12) being smaller than the diameter of the insertion portion (11) and of the anastomose portion (13).

3. Sleeve-type fixing device according to claim 1, wherein the inside of the lower portion of the outer ring (2) has an inwardly protruding ring (21).

4. Sleeve-type fixing device according to claim 2, wherein said insertion portion (11) of said inner ring (1) is provided with axial grooves (112).

5. Sleeve type fixing device according to claim 4, wherein the bottom of the insertion portion (11) is provided with barbs (111).

6. Sleeve-type fixing device according to claim 5, wherein said insertion portion (11) of the inner ring (1) is provided with a flange (121) around the upper portion.

## Patentansprüche

1. Befestigungsvorrichtung in Art einer Manschette für Anastomose für rohrförmige Organe, wie zum Beispiel Därme, Magen, Speiseröhre, mit einem Innenring (1) und einem Außenring (2), wobei der besagte äußere Ring (2) aus einem elastischen Material besteht. Die besagten äußeren (2) und inneren Ringe (1) werden an zwei Enden des Darms (3, 4) genäht und an den beiden Darmenden (3, 4) außen durch den äußeren Ring (2) und den inneren Ring (1) befestigt, **gekennzeichnet durch** die Tatsache, dass der äußere Ring (2) - wenn der Darm (4) an dem betreffenden äußeren Ring (2) befestigt ist - umgeklappt wird, so dass der Darm (4) mit dem Inneren des Außenrings (2), am Außenring (2) und Innenring (1) und mit den Darmanhängen (3, 4) verbunden ist, wenn diese an den Ringen (1, 2) befestigt sind, da alles zusammen geklammert wird.

2. Befestigungsvorrichtung in Art einer Manschette gemäss Anspruch 1, in welcher der obere Teil (13) des Innenringes (1) für die Anastomose verwendet wird, der mittlere Teil (12) für die Verbindung und der untere Teil (11) für die Einführung, wobei der Durchmesser des Verbindungsteils (12) kleiner ist als der Durchmesser des Einführungsteils (11) und des Anastomoseteils (13).

3. Befestigungsvorrichtung in Art einer Manschette gemäss Anspruch 1, bei welcher das Innere des unteren Teils des Außenringes (2) einen Ring (21) hat, der nach Innen ragt.

4. Befestigungsvorrichtung in Art einer Manschette gemäss Anspruch 2, bei welcher der besagte Einführungsteil (11) des besagten Innenrings (1) mit axialen Rillen (112) versehen ist.

5. Befestigungsvorrichtung in Art einer Manschette gemäss Anspruch 4, bei welcher der Boden des Einführungsteils (11) mit Härchen (111) versehen ist.

6. Befestigungsvorrichtung in Art einer Manschette gemäss Anspruch 5, bei welcher der besagte Einfiihrungsteil (11) des Innenrings (1) mit einem Flansch (121) um den oberen Teil herum versehen ist.

## Revendications

1. Dispositif de fixation du type à manchon pour anastomose pour des organes tubulaires, comme par exemple pour intestins, estomac, oesophage, comprenant un anneau interne (1) et un anneau externe (2), dans lequel ledit anneau externe (2) est en matériel élastique, ledit anneau externe (2) et ledit anneau interne (1) sont aptes à être cousus à deux extrémités d'intestins (3, 4) et être fixés aux deux extrémités d'intestins (3, 4) sur la partie externe de l'anneau externe (2) et de l'anneau interne (1), **caractérisé par le fait que** l'anneau externe (2), quand l'intestin (4) est fixé sur ledit anneau externe (2), est apte à être retourné de façon telle que l'intestin (4) soit raccordé à l'intérieur de l'anneau externe (2), l'anneau externe (2) et l'anneau interne (1) et les adventices (3, 4) des intestins, quand ils sont fixés sur les anneaux (1, 2), étant apte à être bridés ensemble.

2. Dispositif de fixation du type à manchon selon la revendication 1, dans laquelle la portion supérieure (13) de l'anneau interne (1) est utilisée pour l'anastomose, la portion médiane (12) est utilisée pour le raccordement, et la portion inférieure (11) est utilisée pour l'insertion, le diamètre de la portion de raccordement (12) étant inférieure au diamètre de la portion d'insertion (11) et de la portion d'anastomose (13).

3. Dispositif de fixation du type à manchon selon la revendication 1, dans laquelle la partie interne de la portion inférieure de l'anneau externe (2) a un anneau (21) en saillie vers l'intérieur.

4. Dispositif de fixation du type à manchon selon la revendication 2, dans laquelle ladite portion d'insertion (11) dudit anneau interne (1) est dotée de rainures axiales (112).

5. Dispositif de fixation du type à manchon selon la revendication 4, dans laquelle le fond de la portion d'insertion (11) est doté d'aspérités (111).

6. Dispositif de fixation du type à manchon selon la revendication 5, dans laquelle ladite portion d'insertion (11) de l'anneau interne (1) est dotée d'une bride (121) autour de la portion supérieure.
